# EUROPEAN PATENT SPECIFICATION

(11) **EP 2 116 188 B1**
(45) Date of publication and mention of the grant of the patent: **16.04.2014**
(21) Application number: 09006225.8
(22) Date of filing: 07.05.2009
(51) Int. Cl.: A61B 8/12

(54) **Ultrasound probe device**
Ultraschallsondenvorrichtung
Dispositif de sonde ultrasonique

(30) Priority: 08.05.2008 JP 2008122533
(43) Date of publication of application: 11.11.2009
(73) Proprietor: Olympus Medical Systems Corp., Tokyo 151-0072 (JP)
(72) Inventor: Nishina, Kenichi, Tokyo 151-0072 (JP); Nakazato, Takeharu, Tokyo 151-0072 (JP)
(74) Representative: von Hellfeld, Axel

(56) References cited:
- EP-A1- 1 759 627
- US-A1- 2004 073 089
- US-A1- 2004 077 926
- US-A1- 2008 051 655

## Description

### BACKGROUND OF THE INVENTION

### 1. Field of the Invention

The present invention relates to an ultrasound probe device including an insertion section having an ultrasound probe, and a hood for the ultrasound probe device fitted for the ultrasound probe device.

### 2. Description of the Related Art

As a device for obtaining an ultrasound tomographic image of an inside of a living body, an ultrasound probe device formed by including an ultrasound probe transmitting and receiving ultrasound at an insertion section to be inserted into a body is used.

As one example of such an ultrasound probe device, there is known an ultrasound endoscope which is formed by being provided with an ultrasound probe at a distal end portion of an insertion section, and obtains an ultrasound tomographic image of a target region by causing the ultrasound probe to abut on the target region in the body as disclosed in, for example, Japanese Patent Publication No. 3504396 .

In the ultrasound probe device of the mode of obtaining the ultrasound tomographic image of the target region by causing an ultrasound probe to abut on the target region in a body, when a force in order to press the ultrasound probe against the target region is applied to cause the ultrasound probe to abut on the target region without a gap, the relative positional relation of the ultrasound probe and the target region sometimes changes due to the action of the force depending on the surface state of the target region.

US 2008/051655 discloses an ultrasound endoscope having a concave ultrasound transducer portion and an optical observation system. The transducer and optical system are disposed such that the focal point of the ultrasound waves and the focal point of the observation optical system are the same. A forceps channel is disposed between the transducer and the optical system so that the instrument can be observed by both the optical system and the ultrasound transducer. In another embodiment, the ultrasound endoscope system comprises a transparent flexible cap adapted to be fitted over the ultrasound transducer and the observation optical system. The cap is intended to come into contact with a body wall and to deform slightly under pressure. The cap may be cylindrical, in which case a transparent liquid is injected into a space defined by the cap, the endoscope, and the body wall, or the cap may a solid transparent block. When the cap is cylindrical, the endoscope is adapted to apply negative pressure to the cap when pressed against a body wall such that the bodily tissue is sucked into the cap close to the surface of the endoscope, and a treatment instrument may be ejected from a forceps channel to treat the tissue inside the cylindrical cap. This embodiment forms the closest prior art for the present invention.

EP 1 759 627 discloses an endoscope having a cylindrical endoscope cap for maintaining a distance between an object to be observed and a distal end of the endoscope, or to suppress movement of the object caused by pulsation of the internal organ.

US 2004/077926 discloses a cylindrical hood body attached to an endoscope. The distal end of the cylindrical hood body is cut at an angle different from perpendicular to an insertion axis.

US2004/073089 discloses an elastically deformable, cylindrical, distal hood component for attachment to an endoscope to regulate a distance between a target and the endoscope so that the target is within the visual field.

The present invention has been made in view of the above described problem, and has an object to provide an ultrasound probe device with a hood section which makes it possible to cause an ultrasound probe to abut on a target region stably.

### SUMMARY OF THE INVENTION

An ultrasound probe device according to claim 1 is provided.

The objects, features and advantages of the invention will become more clearly understood from the following description referring to the accompanying drawings.

### BRIEF DESCRIPTION OF THE DRAWINGS

Fig. 1 is a view explaining an entire configuration of an ultrasound probe device;
Fig. 2 is a view explaining a configuration of a distal end portion of an insertion section of the ultrasound probe device;
Fig. 3 is a view explaining a shape of a hood section of a first embodiment not forming part of the invention;
Fig. 4 is a view showing a state in which the hood section is caused to abut on a target region;
Fig. 5 is a view showing a state in which the target region is sucked into the hood section;
Fig. 6 is a view showing a shape of a hood section of a second embodiment forming part of the invention;
Fig. 7 is a view showing a shape of a hood section of a modified example of the second embodiment not forming part of the invention;
Fig. 8 is a view showing a shape of a hood section of a third embodiment not forming part of the invention;
Fig. 9 is a view of a state in which a target region is sucked by the hood section of the third embodiment;
Fig. 10 is a view showing a shape of a hood section of a fourth embodiment not forming part of the invention;
Fig. 11 is a view showing a state in which the hood section of the fourth embodiment is contracted;
Fig. 12 is a view showing the hood section of a modified example of the fourth embodiment;
Fig. 13 is a view showing a state in which the hood section of the modified example of the fourth embodiment is housed;
Fig. 14 is an explanatory view showing a schematic configuration of an ultrasound probe device of a fifth embodiment;
Fig. 15 is a perspective view showing a configuration of a distal end portion of an insertion section of the ultrasound probe device of the fifth embodiment;
Fig. 16 is a flowchart explaining a sixth embodiment; and
Fig. 17 is a flowchart explaining the sixth embodiment.

### DETAILED DESCRIPTION OF THE PREFERRED EMBODIMENTS

Preferred embodiments of a hood for an ultrasound probe device and the ultrasound probe device of the present invention and embodiments not forming part of the invention will be described with reference to the drawings. In each of the drawings used for the following description, the scale is made to differ for each of the components so that each of the components has such a size to be recognizable on the drawings, and the present invention is not limited only to the numbers and quantities of the components, shapes of the components, the ratios of the sizes of the components, and relative positional relationship of the respective components, which are illustrated in these drawings.

### (First Embodiment)

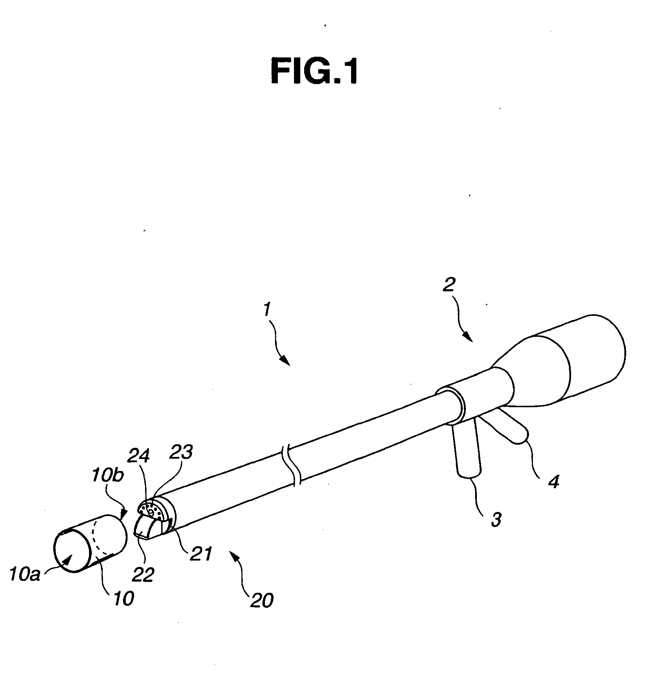

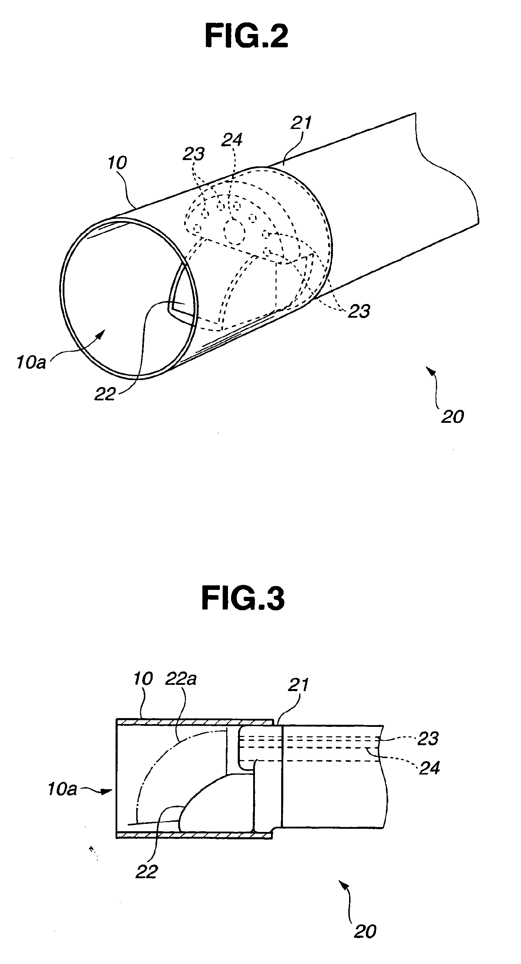

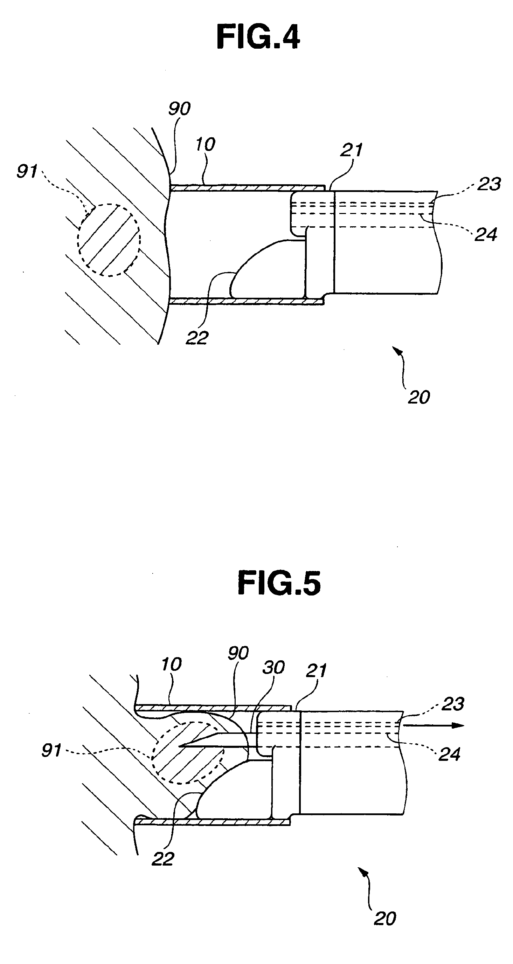

Hereinafter, a first embodiment not forming part of the present invention will be described. Fig. 1 is a view explaining an entire configuration of an ultrasound probe device. Fig. 2 is a view explaining a configuration of a distal end portion of an insertion section of the ultrasound probe device. Fig. 3 is a view explaining a shape of a hood section of the first embodiment. Fig. 4 is a view showing a state in which the hood section is caused to abut on a target region. Fig. 5 is a view showing a state in which the target region is sucked into the hood section.

An ultrasound probe device 1 of the present embodiment transmits and receives ultrasound by an ultrasound probe 22 in a state in which an insertion section 20 is inserted into a body of a subject in order to obtain an ultrasound tomographic image of a predetermined region of the subject.

The ultrasound probe device 1 includes the insertion section 20 to be inserted into a body, and a grasping section 2 which is provided to connect to the insertion section 20 and is grasped by an operator, as shown in Fig. 1. Further, the ultrasound probe 22 transmitting and receiving ultrasound is placed at a distal end portion 21 at a side opposite from a side provided with the grasping section 2 of the insertion section 20.

Hereinafter, in the ultrasound probe device 1, an axis which extends to a side of the distal end portion 21 of the insertion section 20 from a side of the grasping section 2, and is along the insertion section 20, will be called an insertion axis, and a side toward the distal end portion 21 from the grasping section 2 along the insertion axis will be called a distal end side, whereas a side opposite from the distal end side will be called a proximal end side.

In the present embodiment, the ultrasound probe 22 is of a mode which is generally called a convex type, and has a scanning range 22a of ultrasound in a substantially sector shape from a distal end direction of the insertion axis to a side in a plane including the insertion axis as shown in Fig. 3.

The mode of the ultrasound probe 22 is not limited to that of the present embodiment, but may be of the mode of a linear type with, for example, a plurality of ultrasound transducers being arranged in a line on a plane. By the linear type ultrasound probe, for example, the scanning range of ultrasound in a substantially sector shape as in the present embodiment also can be obtained by performing, so-called sector scanning. Further, it goes without saying that the shape and the direction of the scanning range of the ultrasound probe 22 are not limited to those of the present embodiment.

A cylindrical hood section 10 which has an opening portion 10a at a distal end side and surrounds the ultrasound probe 22 is placed at the distal end portion 21 of the insertion section 20 as shown in Fig. 2. The hood section 10 has a cylindrical shape in which the opening portion 10a and an opening portion 10b are formed respectively at the distal end side and a proximal end side as shown in Fig. 1, and the opening portion 10b at the proximal end side has a shape which can be fitted onto an outer peripheral surface portion of the distal end portion 21 of the insertion section 20, in the present embodiment.

Namely, in the present embodiment, the hood section 10 is made attachable to and detachable from the insertion section 20 as a separate member from the insertion section 20 as the hood for an ultrasound probe device. As a result of the hood section 10 being configured to be attachable to and detachable from the insertion section 20 like this, washing and disinfection of the ultrasound probe device 1 can be easily carried out. The hood section 10 may be formed integrally with a member configuring the distal end portion 21 of the insertion section 20.

The hood section 10 is preferably configured such that in at least a region to which ultrasound transmitted from the ultrasound probe 22 is irradiated, that is, a region which is laid on the scanning range 22a, a reflectivity of the ultrasound becomes 25% or less.

Owing to the reflectivity of ultrasound of the hood section 10 being 25% or less, occurrence of artifact which is an image other than the ultrasound tomographic image of a subject, which occurs as a result of the ultrasound reflected by the hood section 10 being received by the ultrasound probe 22, can be suppressed.

The material configuring the hood section 10 is not especially limited, but if the hood section 10 is configured by one or a plurality of kinds of materials of, for example, polyethylene, polymethyl pentene, silicon rubber, nylon and the like, the reflectivity of ultrasound can be made 25% or less.

Further, the shape of the hood section 10 is not especially limited, but if the thickness of the hood section 10 is made an integral multiple of one quarter of the wavelength of the ultrasound transmitted by the ultrasound probe 22 so that the ultrasound reflected in an inner surface of the hood section 10, for example, and the ultrasound reflected in an outer surface of the hood section 10 have opposite phases to each other and cancel out each other, the reflectivity of the hood section 10 can be reduced, which is preferable.

Further, a treatment instrument insertion channel 24 and a suction channel 23 are formed in the distal end portion 21 of the insertion section 20, as the channels which communicate with a space surrounded by the hood section 10.

The treatment instrument insertion channel 24 communicates with a treatment instrument insertion port 4 provided in the grasping section 2 in the proximal end side of the ultrasound probe device 1. By inserting a treatment instrument such as a puncture needle into the treatment instrument insertion port 4, a treatment instrument can be led out into the space surrounded by the hood section 10 through the treatment instrument insertion channel 24. The treatment instrument insertion channel 24 is preferably disposed so that the treatment instrument which is led out into the hood section 10 through the treatment instrument insertion channel 24 can be guided into the scanning range 22a of ultrasound.

Further, the suction channel 23 communicates with a suction connector 3 provided in the grasping section 2 in the proximal end side of the ultrasound probe device 1. A suction device not illustrated is connected to the suction connector 3, and an operator can perform a suction operation for decompressing the inside of the space surrounded by the hood section 10 through the suction channel 23 by operating the suction device.

Further, at least one of the suction channel 23 and the treatment instrument insertion channel 24 is connected to an air-supply and water-supply device not illustrated, and in the present embodiment, at least one of gas and liquid can be supplied into the space surrounded by the hood section 10 through the suction channel 23 or the treatment instrument insertion channel 24 in accordance with the operation or the operator.

The treatment instrument insertion channel 24 and the suction channel 23 may be in the mode of making one channel which communicates with the space surrounded by the hood section 10 to serve a double purpose, or may be in the mode in which a plurality of channels are formed for each of the channels 24 and 23.

According to the ultrasound probe device 1 configured as above, in the state in which the opening portion 10a at the distal end side of the hood section 10 is caused to abut on a target region 90 in which a lesion portion 91, for example, is present in an organ, a digestive tract wall and the like in a subject as shown in Fig. 4, gas inside the space surrounded by the hood section 10 is exhausted through the suction channel 23 and the space is decompressed, whereby the target region 90 can be drawn into the space surrounded by the hood section 10 as shown in Fig. 5.

In this case, the ultrasound probe 22 is placed in the space surrounded by the hood section 10. Therefore, according to the present embodiment, the target region 90 can be caused to abut on the ultrasound probe 22, and observation of the target region 90 by ultrasound tomographic image can be stably performed.

Though not illustrated, in the state in which the opening portion 10a at the distal end side of the hood section 10 is caused to abut on the target region 90 as shown in Fig. 4, an ultrasound medium such as physiological saline is fed into the space surrounded by the hood section 10 through the suction channel 23, after which, the gas inside the space surrounded by the hood section 10 is exhausted and the space can be decompressed.

In this case, even when the target region 90 is a region which is difficult to deform or a region which is hardened due to lesion, and it is difficult to cause the target region 90 to abut on the ultrasound probe 22 because the target region 90 cannot be drawn into the hood section 10 only by decompression, ultrasound can be transmitted to and received from the target region 90 via the ultrasound medium filled in the hood section 10, and therefore, it is more preferable.

Other than the case in which the ultrasound probe 22 is caused to abut on the target region 90, when treatment is performed for the lesion portion 91 of the target region 90 with use of a treatment instrument such as a puncture needle, for example, a relative position of the treatment instrument with respect to the target region 90 changes by the action of the force to press the treatment instrument against the target region.

However, according to the present embodiment, as shown in Fig. 5, after it is checked by the ultrasound tomographic image that the target region 90 including the lesion portion 91 is drawn into the hood section 10, a puncture needle 30 as an example of the treatment instrument is led out into the space surrounded by the hood section 10 through the treatment instrument insertion channel 24, whereby treatment can be stably performed without changing the position of the target region 90 and the treatment instrument.

### (Second Embodiment)

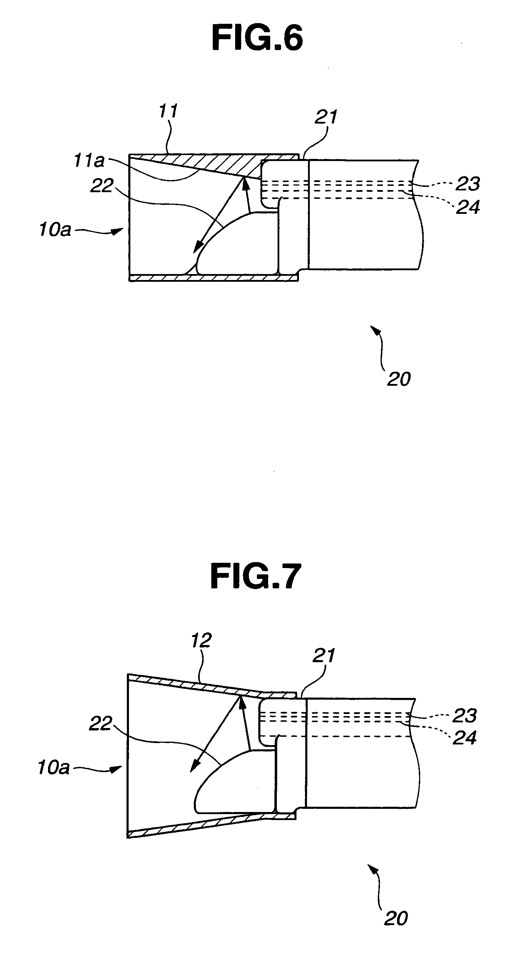

Hereinafter, a second embodiment forming part of the present invention will be described. The second embodiment shows an example of another mode of the shape of the hood section. Fig. 6 is a view showing the shape of the hood section of the second embodiment. Fig. 7 is a view showing a shape of a hood section of a modified example of the second embodiment, but not forming part of the invention.

The second embodiment differs from the above described first embodiment in the shape of the hood section. Therefore, only the difference will be described hereinafter. The same components as those in the first embodiment are assigned with the same reference numerals and characters, and description thereof will be properly omitted.

As shown in Fig. 6, in a hood section 11 of the present embodiment, an inner surface 11a of at least a region to which ultrasound transmitted from the ultrasound probe 22 is irradiated inclines with respect to the insertion axis so as to be closer to the ultrasound probe 22 toward a proximal end side. Further, the hood section 11 is formed so that a thickness of at least the region to which ultrasound transmitted from the ultrasound probe 22 is irradiated becomes larger toward the proximal end side.

An angle of the inclination of the inner surface 11a of the hood section 11 is preferably such an angle that the ultrasound transmitted from the ultrasound probe 22 does not return to the ultrasound probe 22 when the ultrasound specularly reflects at the inner surface 11a.

The inner surface 11a of the hood section 11 is given the inclination to be closer to the ultrasound probe 22 toward the proximal end side like this, and thereby, occurrence of artifact which is an image other than the ultrasound tomographic image of a subject, and generates as a result of the ultrasound reflected by the inner surface 11a being received by the ultrasound probe 22 can be suppressed.

Further, the inclination of the inner surface 11a of the hood section 11 is formed by making the thickness of the corresponding spot of the hood section 11 larger in an inner direction toward the proximal end side, and therefore, an outside diameter of the hood section 11 does not become large due to the inclination of the inner surface 11a being provided.

The configuration in which the inner surface of the region to which the ultrasound transmitted from the ultrasound probe 22 is irradiated, of the hood section is inclined to be closer to the ultrasound probe 22 toward the proximal end side is not limited to the above described configuration, and may be the mode shown as a modified example in Fig. 7, for example, but not forming part of the invention.

In the modified example shown in Fig. 7, a hood section 12 has a so-called tapered inner shape which extensively opens toward a distal end side. In such a mode, the inner surface of the hood section 12 can be given an inclination closer to the ultrasound probe 22 toward the proximal end side, and occurrence of artifact which is an image other than the ultrasound tomographic image of a subject, which occurs as a result of the ultrasound reflected by the inner surface being received by the ultrasound probe 22 can be suppressed.

Further, according to the modified example shown in Fig. 7, the inner surface of the hood section 12 has the same tapered shape in the entire circumference around the insertion axis. Therefore, when the hood section 12 is fitted onto the distal end portion 21 of the insertion section 20, positioning in the rotational direction around the insertion axis of the hood section 12 is not required, and an operation becomes easy.

### (Third Embodiment)

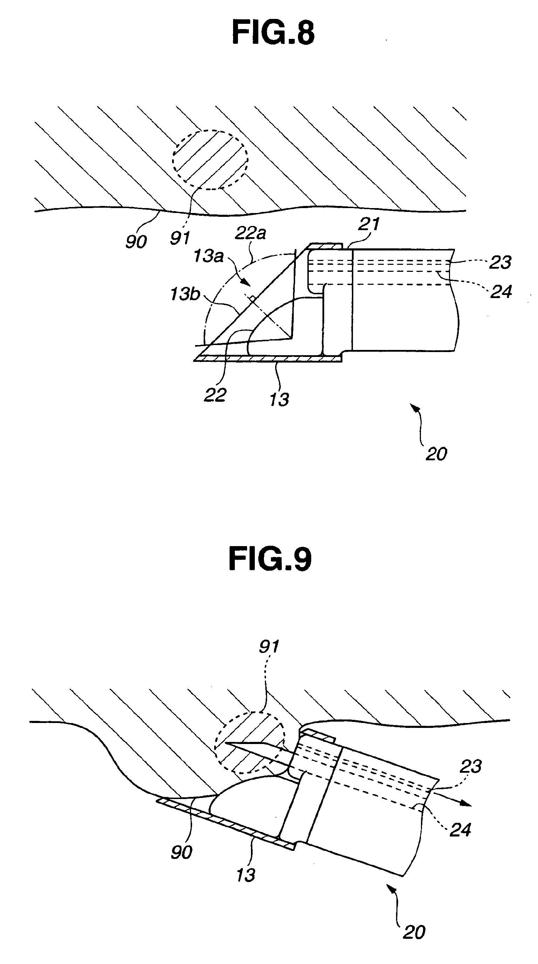

Hereinafter, a third embodiment not forming part of the present invention will be described. The third embodiment shows an example of another mode of a shape of a hood section. Fig. 8 is a view showing the shape of the hood section of the third embodiment. Fig. 9 is a view showing the state in which a target region is sucked by the hood section of the third embodiment.

The third embodiment differs from the above described first embodiment in the shape of the hood section. Therefore, only the difference will be described hereinafter. The same components as those in the first embodiment are assigned with the same reference numerals and characters, and description thereof will be properly omitted.

As shown in Fig. 8, in a hood section 13 of the present embodiment, an opening portion 13a at a distal end side has a shape cut by a plane 13b having a predetermined angle with respect to an insertion axis. As a result, the opening portion 13a of the hood section 13 is formed to include a region to which ultrasound transmitted from the ultrasound probe 22 is irradiated. In other words, in the hood section 13 of the present embodiment, the opening portion at the distal end side is formed to be in a shape cut by the plane forming the predetermined angle with respect to the side surface in the above described cylindrical shape.

Namely, the present embodiment is configured so that the hood section 13 is not laid on the scanning range 22a of ultrasound of the ultrasound probe 22. Further, the hood section 13 includes the opening portion 13a at the distal end side, and therefore, the target region can be sucked into the space surrounded by the hood section 13 as in the above described first embodiment.

The present embodiment has such a configuration, and has nothing to block the scanning range 22a of ultrasound of the ultrasound probe 22. Therefore, a clearer ultrasound tomographic image can be obtained in addition to the same effect as that of the above described first embodiment.

Further, in the present embodiment, the opening portion 13a at the distal end side of the hood section 13 forms a predetermined angle with respect to the insertion axis, and therefore, even when the angle formed by the insertion axis of the insertion section 20 and a wall surface of the target region 90 is an obtuse angle as shown in Fig. 9, the target region 90 can be easily sucked into the space surrounded by the hood section 13. This is especially effective when the target region is an inner wall surface of a digestive tract which has a relatively small diameter, such as an esophagus or enteric canal, a trachea, a blood vessel or the like, in which the degree of freedom of the position and posture of the ultrasound probe device 1 is low.

### (Fourth Embodiment)

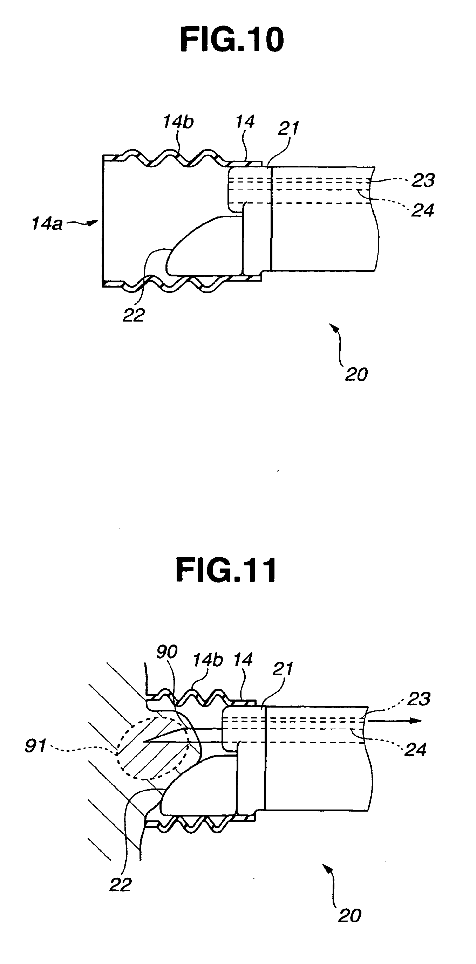

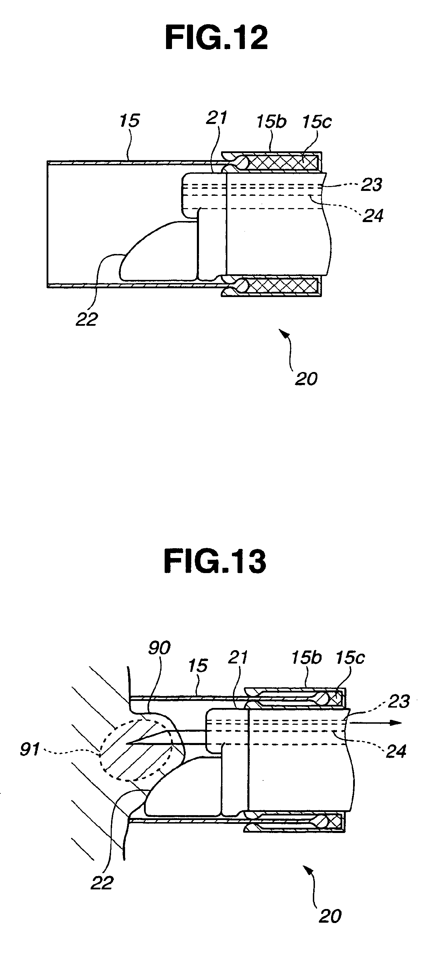

Hereinafter, a fourth embodiment not forming part of the present invention will be described. The fourth embodiment shows an example of another mode of the shape of the hood section. Fig. 10 is a view showing the shape of the hood section of the fourth embodiment. Fig. 11 is a view showing the state in which the hood section of the fourth embodiment is contracted. Fig. 12 is a view showing a hood section of a modified example of the fourth embodiment. Fig. 13 is a view showing a state in which the hood section of the modified example of the fourth embodiment is housed.

The fourth embodiment differs from the above described first embodiment in the shape of the hood section. Therefore, only the difference will be described hereinafter. The same components as those in the first embodiment are assigned with the same reference numerals and characters, and description thereof will be properly omitted.

As shown in Figs. 10 and 11, a hood section 14 of the present embodiment has a cylindrical shape having an opening portion 14a at a distal end side, and has a configuration contractible in the direction along the insertion axis.

The configuration of making the hood section 14 contractible is not especially limited, but in the present embodiment, as an example, the hood section 14 is configured by an elastically deformable resin material, and a side surface portion 14b is formed into a bellows shape, whereby the hood section 14 is elastically contracted in the insertion axis direction. The hood section may be of a mode contracting in a diameter direction instead of the insertion axis direction.

The present embodiment has such a configuration. Therefore, gas inside the space surrounded by the hood section 14 is exhausted through the suction channel 23 and the space is decompressed in the state in which an opening portion 14a at a distal end side of the hood section 14 is caused to abut on the target region 90, whereby the target region is drawn into the hood section 14, and the hood section 14 is contracted in the insertion axis direction as shown in Fig. 11.

Thereby, even when the target region 90 is a region which is difficult to deform or a region which is hardened by lesion, the target region 90 can be caused to abut on the ultrasound probe 22.

Further, a contraction amount in the insertion axis direction of the hood section 14 changes in accordance with the decompression state inside the space surrounded by the hood section 14, and therefore, the contraction amount can be properly changed. Therefore, a distance between the target region 90 and the ultrasound probe 22, or a distance between the target region 90 and the distal end portion 21 can be made to be an optimal value for observation or treatment, and operability is enhanced.

For example, after observation of the target region 90 is performed by the ultrasound probe 22, and the detailed positioning of the lesion portion 91 is identified, the distance between the target region 90 and the distal end portion 21 can be increased until the optimal space for treatment by the treatment instrument is obtained without change in the position of the lesion portion 91 and the treatment instrument insertion channel 24 with respect to the direction orthogonal to the insertion axis. Thus, the treatment instrument can be positioned to the lesion portion 91 of the target region 90 accurately and easily.

Besides the configuration which contracts the hood section in the axial direction, even when a hood section 15 is configured to be capable of advancing and retreating movement in the axial direction as in the modified example of the present embodiment shown in Figs. 12 and 13, the same effect can be obtained.

In the modified example of the present embodiment shown in Figs. 12 and 13, a cylinder section 15b in a cylindrical shape is placed at an outer circumference of the distal end portion 21 of the insertion section 20, and the hood section 15 is inserted with a proximal end side as a piston portion slidable in the insertion axis direction inside the cylinder section 15b.

A working fluid 15c is filled in a space closed by the proximal end side of the hood section 15 and the cylinder section 15b. If a compressible fluid is adopted as the working fluid 15c, the working fluid 15c works as a spring, and therefore, the same effect as the configuration which contracts the hood section in the axial direction is obtained.

Further, as compared with the configuration which contracts the hood section as in the above described embodiment, the modified example of the present embodiment is preferable because due to favorable rectilinearity of advancing and retreating movement of the hood section 15, positioning can be reliably performed, and operability is enhanced.

In the modified example of the present embodiment shown in Figs. 12 and 13, such a configuration may be adopted that a compressible or non-compressible fluid is used as the working fluid 15c, the space housing the working fluid 15c of the cylinder section 15b is caused to communicate with the grasping section 2 side by piping not illustrated placed in the insertion section, and the pressure inside the cylinder section 15b is made controllable.

According to such a configuration, the advancing amount of the hood section 15 in the insertion axis direction can be changed in accordance with the pressure inside the cylinder section 15b, and positioning of the target region 90 and the distal end portion 21 of the insertion section 20 can be performed more precisely.

Further, when the ultrasound probe device 1 is inserted into a subject, the pressure inside the cylinder section 15b is reduced, whereby the hood section 15 is housed and the length in the insertion axis direction of the distal end portion 20 can be shortened. Therefore, operability at the time of insertion is enhanced.

In the above described first to fourth embodiments, the hood sections differing in shape respectively are described, but it goes without saying that the hood sections are attachable to and detachable from the insertion sections of the ultrasound probe devices, and therefore, the hood sections are properly replaceable in accordance with the regions and treatment contents for which the ultrasound probe devices are used.

### (Fifth Embodiment)

A fifth embodiment forming part of the present invention will be described hereinafter. The fifth embodiment is another example of an ultrasound probe device to which the hood sections of the above described embodiments are applicable.

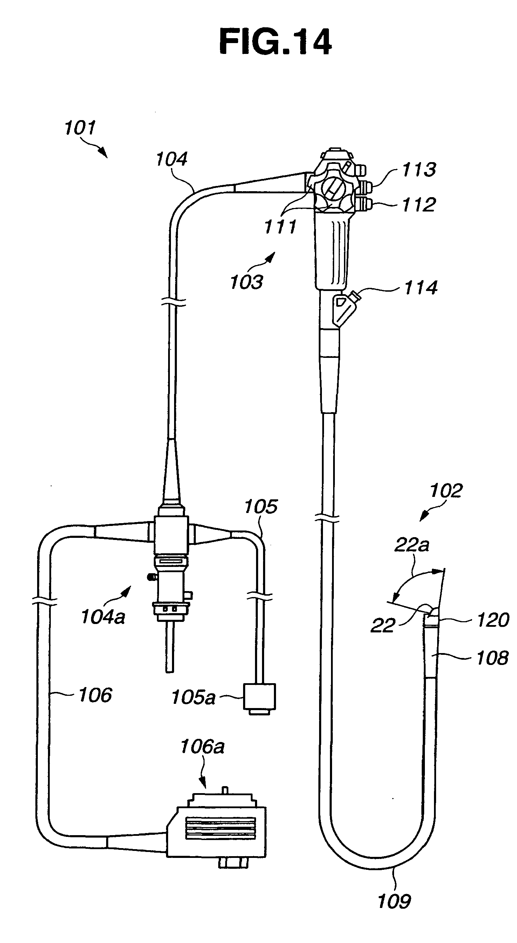

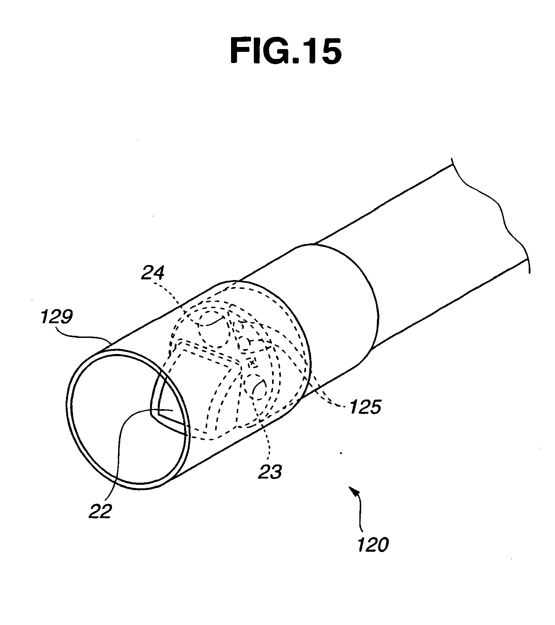

Fig. 14 is an explanatory view showing a schematic configuration of an ultrasound probe device of the fifth embodiment. Fig. 15 is a perspective view showing a configuration of a distal end portion of the insertion section of the ultrasound probe device of the fifth embodiment.

The fifth embodiment differs from the ultrasound probe devices of the above described first to fourth embodiments in the constitution having an optical observation system at the insertion section. Therefore, only the difference will be described hereinafter. The same components as those in the first embodiment are assigned with the same reference numerals and characters, and description thereof will be properly omitted.

An ultrasound probe device 101 of the present embodiment is mainly configured by an insertion section 102 which is inserted into a body of a subject, an operation section 103 located at a proximal end of the insertion section 102, and a universal cord 104 extended from a side portion of the operation section 103.

An endoscope connector 104a connected to a light source device not illustrated is provided at a proximal end portion of the above described universal cord 104. From the endoscope connector 104a, an electric cable 105 which is attachably and detachably connected to a camera control unit not illustrated through an electric connector 105a, and an ultrasound cable 106 which is attachably and detachably connected to an ultrasound observation device not illustrated via an ultrasound connector 106a are extended.

The above described insertion section 102 is configured by connecting in sequence from a distal end side, a distal end portion 120, a bending section 108 which is located at a proximal end side of the distal end portion 120 and bendable, and a flexible tube section 109 which is located at a rear end of the bending section 108 to extend to a distal end portion of the above described operation section 103, is small in diameter and elongated, and has flexibility. Further, the ultrasound probe 22 for transmitting and receiving ultrasound is provided at the distal end side of the above described distal end portion 120.

The above described operation section 103 is provided with an angle knob 111 which controls the above described bending section 108 to bend in a desired direction, an air supply and water supply button 112 for performing air supply and water supply operation, a suction button 113 for performing a suction operation, a treatment instrument insertion port 114 which is an inlet port of a treatment instrument to be led into a body, and the like.

As shown in Fig. 15, an observation optical system 125 constituted of an illumination lens configuring an illumination optical section for irradiating illumination light to an observation region, and an objective lens configuring an observation optical section capturing an optical image of the observation region is placed at the distal end portion 120 of the insertion section 102.

Further, as in the first embodiment, the ultrasound probe 22 is placed at the distal end portion, and a hood section 129 having a cylindrical shape surrounding the ultrasound probe 22 is provided. The hood section 129 is configured by a light transmitting material which transmits a light with a wavelength in a visible light region at a predetermined transmissivity.

Further, the treatment instrument insertion channel 24 and the suction channel 23 which communicate with the space surrounded by the hood section 129 are formed in the distal end portion 120. The suction channel 23 may be also used as the channel for inserting a treatment instrument through.

As above, in the present embodiment, the hood section 129 is configured by a light transmitting material, and thereby, in the ultrasound probe device 101 including the observation optical system 125 for performing observation of the inside of a body by visible light, the same operation and effect as in the above described embodiments can be obtained. Further, when the observation optical system 125 is included in the ultrasound probe device 101, the observation optical system 125 and the treatment instrument insertion path are preferably disposed so that the treatment instrument which passes through the treatment instrument insertion path is within the field of view of the observation optical system 125.

### (Sixth Embodiment)

With use of the hood for an ultrasound probe device or the ultrasound probe device described in any of the above described first to fifth embodiments, the manipulation described as follows can be performed.

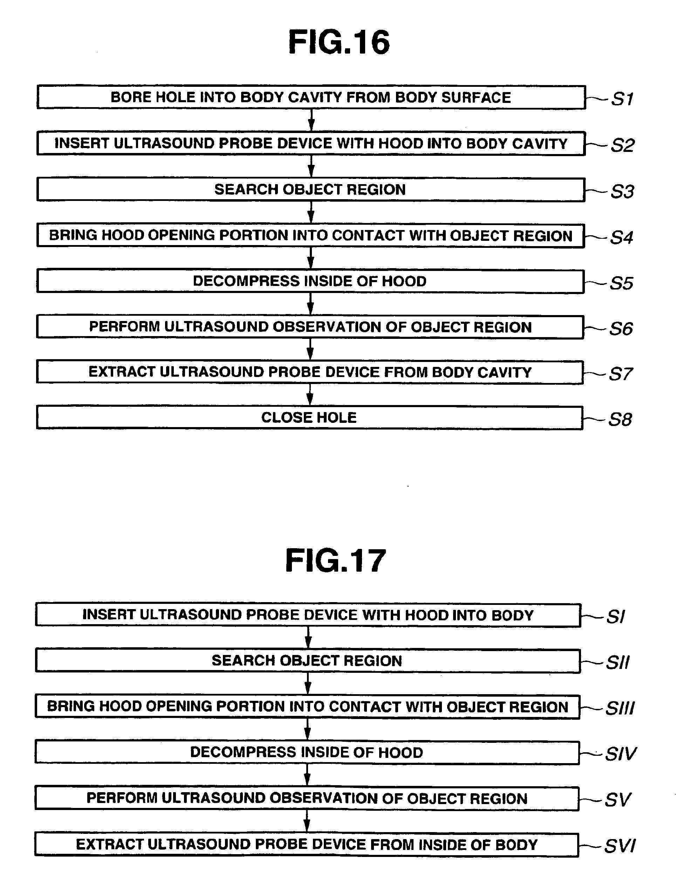

Fig. 16 shows a flow relating to the manipulation in the case of approaching from outside a body with use of the ultrasound probe device as an example. As shown in Fig. 16, the present embodiment includes at least a step (S1) of boring a hole into a body cavity from a body surface, a step (S2) of inserting the ultrasound probe device with a hood into the body cavity, a step (S3) of searching an object region, a step (S4) of bringing a hood opening portion into contact with the object region, a step (S5) of decompressing an inside of the hood, a step (S6) of performing ultrasound observation of the object region, a step (S7) of extracting the ultrasound probe device from the body cavity, and a step (S8) of closing the hole. Hereinafter, the details of the respective steps will be described.

### (S1)

S1 is the step of boring a hole into a body cavity from a body surface. The method for boring a hole is not especially limited, the conventionally known method can be used, and, for example, a surgical knife, or an electric knife can be used. Further, a hole may be bored by insertion of a trocar in accordance with the purpose.

In step S1, after the hole is bored, inert gas may be introduced into the body cavity. When the intended observation region is a lung, a hole is preferably bored between the ribs.

### (S2)

S2 is the step of inserting the ultrasound probe device with the hood into a body cavity. The hood mentioned here means the hood for the ultrasound probe device described in any of the above described first to fifth embodiments, and may be attachable and detachable with respect to the ultrasound probe device, or may be formed integrally with the ultrasound probe device.

Further, in step S2, the observation system other than the ultrasound probe device may be inserted into a body cavity. For example, an endoscope capable of visible light observation, fluorescent observation, or infrared observation may be inserted. In this case, dissection for an endoscope may be performed separately from that for the ultrasound probe device in step S1.

### (S3)

S3 is the step of searching an object region. Search for the object region may be performed by using the ultrasound probe device, or may be performed by using an observation system other than the ultrasound probe device when the observation system other than the ultrasound probe device is used, or may be performed with use of both of the ultrasound probe device and the observation system.

### (S4)

S4 is the step of bringing the hood opening portion into contact with the object region. Since the inside of the hood is decompressed in S5 which is the next step, the entire circumference of the hood opening portion is preferably in contact with a living body.

### (S5)

S5 is the step of decompressing the inside of the hood. A living tissue including the object region is drawn into the hood by decompression of the inside of the hood, and the object region can be brought into contact with the ultrasound probe of the ultrasound probe device. The method for decompressing the inside of the hood is not especially limited, but when a gas supply and water supply conduit line is provided in the ultrasound probe device, it can be used.

### (S6)

S6 is the step of performing ultrasound observation of the object region. In S5 which is the previous step, the living tissue is drawn into the hood by decompression, and therefore, ultrasound observation can be stably performed. Further, in step S6, an ultrasound medium may be introduced into the hood. The ultrasound medium is not especially limited, and, for example, physiological saline, water or the like can be used.

Further, treatment may be performed while observation is performed. Puncture and extraction of the tissue may be performed with use of a puncture needle while observation is performed, for example, or injection of a medicine may be performed with use of a puncture needle, or extraction of the tissue may be performed with use of grasping forceps, or the tissue may be cut off with use of a radio frequency snare.

### (S7)

S7 is the step of extracting the ultrasound probe device from the body cavity.

### (S8)

S8 is the step of closing the hole. The method for closing the incised spot is not especially limited, and the conventionally known method can be used.

Fig. 17 shows a flow relating to the procedure of the manipulation in the case of approaching from the inside of a lumen with use of the ultrasound probe device. As shown in Fig. 17, the present embodiment includes at least a step (SI) of inserting an ultrasound probe device with a hood into a body, a step (SII) of searching an object region, a step (SIII) of bringing a hood opening portion into contact with the object region, a step (SIV) of decompressing the inside of the hood, a step (SV) of performing ultrasound observation of the object region, and a step (SVI) of extracting the ultrasound probe from the inside of the body. Hereinafter, the details of the respective steps will be described.

### (SI)

SI is the step of inserting the ultrasound probe device with the hood into a body cavity. The hood mentioned here means the hood for the ultrasound probe device described in any of the above described first to fifth embodiments, and may be attachable and detachable with respect to the ultrasound probe device, or may be formed integrally with the ultrasound probe device. An insertion route of the ultrasound probe device is not especially limited, and can be selected from oral and transanal route, for example, in accordance with the object observation region.

### (SII)

SII is the step of searching an object region.

### (SIII)

SIII is the step of bringing the hood opening portion into contact with the object region. Since the inside of the hood is decompressed in SIV which is the next step, the entire circumference of the hood opening portion is preferably in contact with a living body.

### (SIV)

SIV is the step of decompressing the inside of the hood. A living tissue including the object region is drawn into the hood by decompression of the inside of the hood, and the object region can be brought into contact with the ultrasound probe of the ultrasound probe device. The method for decompressing the inside of the hood is not especially limited, but when a gas supply and water supply conduit line is provided in the ultrasound probe device, it can be used.

### (SV)

SV is the step of performing ultrasound observation of the object region. In SIV which is the previous step, the living tissue is drawn into the hood by decompression, and therefore, ultrasound observation can be stably performed. Further, in step SV, an ultrasound medium may be introduced into the hood. The ultrasound medium is not especially limited, and, for example, physiological saline, water or the like can be used.

Further, treatment may be performed while observation is performed. Puncture and extraction of the tissue may be performed with use of a puncture needle while observation is performed, for example, or injection of a medicine may be performed with use of a puncture needle, or extraction of the tissue may be performed with use of grasping forceps, or the tissue may be cut off with use of a radio frequency snare.

### (SVI)

SVI is the step of extracting the ultrasound probe device from the body cavity.

## Claims

1. An ultrasound probe device (1, 101) comprising:
an insertion section (20, 102) having an ultrasound probe (22); and
a hood section (11, 129), which has a cylindrical shape having opening portions (10a, 10b) at both ends and which surrounds the ultrasound probe (22) when fitted to the insertion section (20, 102) in the opening portion (10b) at a proximal end side of the cylindrical shape,
**characterized in that**, in a state fitted to the insertion section (20, 102), a thickness of the cylindrical shape of the hood section (11, 129) increases toward the opening portion (10b) at the proximal end side such that an inner surface (11a) of the cylindrical shape in a region (22a) to which ultrasound transmitted from the ultrasound probe (22) is irradiated inclines to be closer to the ultrasound probe (22) toward the opening portion (10b) at the proximal end side, wherein an angle of inclination of the inner surface (11a) of the hood section (11,129) in the region to which ultrasound is irradiated is such that ultrasound transmitted from the ultrasound probe (22) does not return to the ultrasound probe (22) when the ultrasound specularly reflects at the inner surface (11a); and
wherein a cross-section of the hood portion has:
a first portion having the inclined inner surface (11a) that is inclined from a distal end of the hood section (11, 129) to a distal end of the insertion section (20, 102); and
a second portion having a thickness that is constant from the distal end to the proximal end of the hood section (11, 129) and that is not inclined.

2. The ultrasound probe device (1, 101), according to any preceding claim, wherein a space surrounded by the hood section (11, 129) is capable of being decompressed.

3. The ultrasound probe device according to claim 2, wherein the hood section (11, 129) is attachable to and detachable from the insertion section (20, 102).

4. The ultrasound probe device according to one of claims 2 to 3, wherein the hood section (11, 129) is formed so that the opening portion (10a) at the distal end side is adapted to include a target region (90) to which ultrasound transmitted from the ultrasound probe (22) is irradiated.

5. The ultrasound probe device according to one of claims 2 to 4, wherein the insertion section (20, 102) is capable of housing the hood section (11, 129).

6. The ultrasound probe device according to one of claims 2 to 5, wherein the ultrasound probe (22) is of a convex type or a linear type.

7. The ultrasound probe device according to one of claims 2 to 6, wherein the insertion section (20, 102) comprises at least one channel (23, 24) communicating with a space surrounded by the hood section (11, 129).

8. The ultrasound probe device according to claim 7, wherein at least two of the channels (23, 24) are included,
at least one of the channels (24) is a channel for inserting a treatment instrument, and
at least one of the channels (23) is a channel for suction.

9. The ultrasound probe device according to one of claims 2 to 8, wherein the insertion section (20, 102) comprises an optical observation system (125).

10. The ultrasound probe device according to claim 9, wherein the hood section (11, 129) is formed of a material adapted to transmit visible light.

11. The ultrasound probe device according to any preceding claim, wherein the ultrasound probe has a scanning range (22a) of ultrasound in a sector shape extending from a direction of an insertion axis along the insertion section to a side in a plane comprising the insertion axis, and wherein the region of the hood section (11, 129) to which ultrasound is transmitted is a region laid on the scanning range (22a).

12. The ultrasound probe device according to any preceding claim, wherein the first portion of the hood section (11, 129) is configured to abut a side of the ultrasound probe (22) opposite to a side of the ultrasound probe (22) from which ultrasound is irradiated.

## Patentansprüche

1. Ultraschallsondengerät (1, 101) umfassend:
einen Einführbereich (20, 102), der eine Ultraschallsonde (20) aufweist; und
einen Abdeckbereich (11, 129), der eine zylindrische Form mit Öffnungsbereichen (10a, 10b) an beiden Enden hat und der die Ultraschallsonde (22) umgibt, wenn er an dem Einführbereich (20, 102) in dem Öffnungsbereich (10b) an einer proximalen Endseite der zylindrischen Form angebracht ist,
**dadurch gekennzeichnet, dass**, in einem an dem Einführbereich (20, 102) angebrachten Zustand, eine Dicke der zylindrischen Form des Abdeckbereichs (11, 129) in Richtung des Öffnungsbereichs (10b) an der proximalen Endseite zunimmt, so dass eine innere Oberfläche (11a) der zylindrischen Form in einem Bereich (22a), der mit Ultraschall, der von der Ultraschallsonde (22) übertragen wird, bestrahlt wird, geneigt ist, um näher an der Ultraschallsonde (22) in Richtung des Öffnungsbereichs (10b) an der proximalen Endseite zu sein, wobei ein Neigungswinkel der inneren Oberfläche (11a) des Abdeckbereich (11, 129) in dem Bereich, der mit Ultraschall bestrahlt wird, so gewählt ist, dass der Ultraschall, der von der Ultraschallsonde (22) übertragen wird, nicht an die Ultraschallsonde (22) zurück übertragen wird, wenn der Ultraschall spiegelnd an der inneren Oberfläche (11a) reflektiert wird; und
wobei ein Querschnitt des Abdeckbereichs umfasst:
einen ersten Abschnitt, der die geneigte inneren Oberfläche (11a) umfasst, die von einem distalen Ende des Abdeckbereichs (11,129) zu einem distalen Ende des Einführbereichs (20, 102) geneigt ist; und
einen zweiten Abschnitt, der eine Dicke hat, die von dem distalen Ende zu dem proximalen Ende des Abdeckbereichs (11,129) konstant ist, und der nicht geneigt ist.

2. Ultraschallsondengerät (1, 101) gemäß einem der vorhergehenden Ansprüche, wobei ein Bereich, der von dem Abdeckbereich (11, 129) umgeben ist, geeignet ist, dekomprimiert zu werden.

3. Ultraschallsondengerät gemäß Anspruch 2, wobei der Abdeckbereich (11, 129) verbindbar mit und trennbar von dem Einführbereich (20, 102) ist.

4. Ultraschallsondengerät (1, 101) gemäß einem der Ansprüche 2 bis 3, wobei der Abdeckbereich (11, 129) so ausgebildet ist, dass der Öffnungsbereich (10a) an der distalen Endseite dazu eingerichtet ist, einen Zielbereich (90) zu umfassen, der mit Ultraschall bestrahlt wird, der von der Ultraschallsonde (22) übertragen wird.

5. Ultraschallsondengerät gemäß einem der Ansprüche 2 bis 4, wobei der Einführbereich (20, 102) geeignet ist, den Abdeckbereich (11, 129) aufzunehmen.

6. Ultraschallsondengerät gemäß einem der Ansprüche 2 bis 5, wobei die Ultraschallsonde (22) von konvexem Typ oder linearem Typ ist.

7. Ultraschallsondengerät gemäß einem der Ansprüche 2 bis 6, wobei der Einführbereich (20, 102) wenigstens einen Kanal (23, 24) umfasst, der mit einem Bereich kommuniziert, der von dem Abdeckbereich (11, 129) umgeben ist.

8. Ultraschallsondengerät gemäß Anspruch 7, wobei wenigstens zwei der Kanäle (23, 24) vorgesehen sind,
wenigstens einer der Kanäle (24) ein Kanal zur Einführung eines Behandlungsinstruments ist, und
wenigstens einer der Kanäle (23) ein Kanal zum Saugen ist.

9. Ultraschallsondengerät gemäß einem der Ansprüche 2 bis 8, wobei der Einführbereich (20, 102) ein optisches Beobachtungssystem (125) umfasst.

10. Ultraschallsondengerät gemäß Anspruch 9, wobei der Abdeckbereich (11, 129) aus einem Material gebildet ist, das geeignet ist, sichtbares Licht zu übertragen.

11. Ultraschallsondengerät gemäß einem der vorhergehenden Ansprüche, wobei die Ultraschallsonde einen Abtastbereich (22a) von Ultraschall in einer Sektorform hat, der sich von einer Richtung einer Einführachse entlang des Einführabschnitts zu einer Seite in einer Ebene erstreckt, die die Einführachse umfasst, und wobei der Bereich des Abdeckbereichs (11, 129), zu dem der Ultraschall übertragen wird, ein Bereich ist, der in dem Abtastbereich (22a) liegt.

12. Ultraschallsondengerät gemäß einem der vorhergehenden Ansprüche, wobei der erste Bereich des Abdeckbereichs (11, 129) so geformt ist, dass er an eine Seite der Ultraschallsonde (22) anstößt, die entgegengesetzt zu einer Seite der Ultraschallsonde (22) liegt, von der der Ultraschall abgestrahlt wird.

## Revendications

1. Dispositif (1, 101) de sonde ultrasonore comprenant :
une section (20, 102) d'insertion ayant une sonde ultrasonore (22) ; et
une section (11,129) de coiffe, qui a une forme cylindrique ayant des parties (10a, 10b) d'ouverture aux deux extrémités et qui entoure la sonde ultrasonore (22) lorsqu'elle est ajustée sur la section (20, 102) d'insertion dans la partie (10b) d'ouverture au niveau d'un côté d'extrémité proximale de la forme cylindrique,
**caractérisé en ce que**, dans un état ajusté sur la section (20, 102) d'insertion, une épaisseur de la forme cylindrique de la section (11, 129) de coiffe augmente vers la partie (10b) d'ouverture au niveau du côté d'extrémité proximale, de telle sorte qu'une surface intérieure (11a) de la forme cylindrique dans une région (22a) sur laquelle un ultrason transmis à partir de la sonde ultrasonore (22) est projeté s'incline pour être plus proche de la sonde ultrasonore (22) vers la partie (10b) d'ouverture au niveau du côté d'extrémité proximale, dans lequel un angle d'inclinaison de la surface intérieure (11a) de la section (11, 129) de coiffe dans la région sur laquelle un ultrason est projeté est tel que l'ultrason transmis à partir de la sonde ultrasonore (22) ne revient pas sur la sonde ultrasonore (22) lorsque l'ultrason est réfléchi de façon spéculaire au niveau de la surface intérieure (11a) ; et
dans lequel une section transversale de la partie de coiffe a :
une première partie ayant la surface intérieure (11a) inclinée qui est inclinée d'une extrémité distale de la section (11, 129) de coiffe jusqu'à une extrémité distale de la section (20, 102) d'insertion ; et
une deuxième partie ayant une épaisseur qui est constante de l'extrémité distale jusqu'à l'extrémité proximale de la section (11, 129) de coiffe et qui n'est pas inclinée.

2. Dispositif (1, 101) de sonde ultrasonore selon l'une des revendications précédentes, dans lequel un espace entouré par la section (11, 129) de coiffe est apte à être décompressé.

3. Dispositif de sonde ultrasonore selon la revendication 2, dans lequel la section (11, 129) de coiffe peut être attachée à et détachée de la section (20, 102) d'insertion.

4. Dispositif de sonde ultrasonore selon l'une des revendications 2 à 3, dans lequel la section (11, 129) de coiffe est formée de telle manière que la partie (10a) d'ouverture au niveau du côté d'extrémité distale est adaptée à inclure une région cible (90) sur laquelle un ultrason transmis à partir de la sonde ultrasonore (22) est projeté.

5. Dispositif de sonde ultrasonore selon l'une des revendications 2 à 4, dans lequel la section (20, 102) d'insertion est apte à loger la section (11, 129) de coiffe.

6. Dispositif de sonde ultrasonore selon l'une des revendications 2 à 5, dans lequel la sonde ultrasonore (22) est d'un type convexe ou d'un type linéaire.

7. Dispositif de sonde ultrasonore selon l'une des revendications 2 à 6, dans lequel la section (20, 102) d'insertion comprend au moins un canal (23, 24) communiquant avec un espace entouré par la section (11, 129) de coiffe.

8. Dispositif de sonde ultrasonore selon la revendication 7, dans lequel au moins deux des canaux (23, 24) sont inclus,
au moins un des canaux (24) est un canal pour insérer un instrument de traitement, et
au moins un des canaux (23) est un canal pour aspiration.

9. Dispositif de sonde ultrasonore selon l'une des revendications 2 à 8, dans lequel la section (20, 102) d'insertion comprend un système optique (125) d'observation.

10. Dispositif de sonde ultrasonore selon la revendication 9, dans lequel la section (11, 129) de coiffe est constituée d'un matériau adapté à transmettre une lumière visible.

11. Dispositif de sonde ultrasonore selon une quelconque revendication précédente, dans lequel la sonde ultrasonore a une plage (22a) de balayage d'ultrason dans une forme de secteur s'étendant d'un sens d'un axe d'insertion le long de la section d'insertion jusqu'à un côté dans un plan comprenant l'axe d'insertion, et dans lequel la région de la section (11, 129) de coiffe sur laquelle un ultrason est transmis est une région reposant sur la plage (22a) de balayage.

12. Dispositif de sonde ultrasonore selon une quelconque revendication précédente, dans lequel la première partie de la section (11, 129) de coiffe est configurée pour venir buter sur un côté de la sonde ultrasonore (22) opposé à un côté de la sonde ultrasonore (22) duquel un ultrason est projeté.
